# EUROPEAN PATENT SPECIFICATION

(11) **EP 1 655 026 B1**
(45) Date of publication and mention of the grant of the patent: **19.08.2009**
(21) Application number: 05076453.9
(22) Date of filing: 22.06.2005
(51) Int. Cl.: A61K 31/222, A61K 31/5415, A61P 29/00

(54) **Solid pharmaceutical formulations comprising diacereine and meloxicam**
Feste pharmazeutische Arzneimittelformulierung enthaltend Diacerein und Meloxicam
Composition pharmaceutique solide comprénant diacerein et meloxicame

(30) Priority: 04.10.2004 MX PA04009698
(43) Date of publication of application: 10.05.2006
(73) Proprietor: World Trade Import-Export, WTIE A.G., 6302 Zug (CH)
(72) Inventor: Garcia Armenta, Maria Elena, 45020 Zapopan, Jalisco (MX); Santos Murillo, Josefina, 45130 Zapopan, Jalisco (MX); Alvarez Ochoa, Victor Guillermo, 46010 Zapopan, Jalisco (MX); Flores Mendoza, Consuelo, 44600 Guadalajara, Jalisco (MX)
(74) Representative: Carvajal y Urquijo, Isabel

(56) References cited:
- WO-A-20/04062552
- WO-A-20/05021041
- US-A1- 2002 128 317
- US-B1- 6 610 750
- CARRABBA M ET AL: "A COMPARISON OF THE LOCAL TOLERABILITY, SAFETY AND EFFICACY OF MELOXICAM AND PIROXICAM SUPPOSITORIES IN PATIENTS WITH OSTEOARTHRITIS: A SINGLE-BLIND, RANDOMIZED, MULTICENTRE STUDY" CURRENT MEDICAL RESEARCH AND OPINION, HANTS, GB, vol. 13, no. 6, 1995, pages 343-355, XP001095128

## Description

Solid pharmaceutical forms are preparations generally disk-shaped, scored and non-scored and in different sizes containing the active principle(s) and additives, which are obtained by compressing powders or granules into tablets, capsules, patches, pessaries, beads, suppositories, troches or lozenges.

### BACKGROUND OF THE INVENTION

ARTHROSIS, also called OSTEOARTHRITIS or JOINT DEGENERATIVE DISEASE, more than an illness, it is a syndrome, the final expression of disturbances produced on the articulation by a diverse group of etiopathogenic processes (excessive or repeating mechanical overload, genetic factors, metabolic or inflammatory processes).

As for economical and social costs, the disease has an enormous importance in the occidental world: it is the most important cause of functional disability regarding processes related to the locomotive apparatus, and the second cause of permanent disability after cardiovascular diseases.

Arthrosis is the most frequent of all joint diseases and its prevalence increases with age. It is estimated that radiological signs of arthrosis are unusual before 40 years of life (2%), they show in 30% of people with an age between 45 and 65 years old and in 68% of people above 65 years old. Typical arthrosis joint disturbances begin in the second decade of life, affecting about 90% of people above 40 years old.

The illness is distributed universally, although there are geographical differences, due in part to genetic factors, environmental correlation and to the different usage of the joints.

Osteoarthritis is the eighth cause of disability worldwide, according to studies realized by WHO.

As for rheumatoid arthritis, this is a disorder with a relatively common prevalence and worldwide distribution affecting adult men and women. It has the potential to severely affect survival, functionality and quality of life of the individual affected with, as well as the ability to keep a satisfactory employment. Mortality due to direct causes or by complications derived from rheumatoid arthritis is still almost twice than observed in the control population, and this trend has kept unchanged for the last four decades. In the Latin-American countries the economical impact derived from this pathology has been underestimated. In the U.S.A. it has been considered as one of the principal reasons for invalidity pension and direct or indirect economical losses.

US-B1-6610750 discloses the therapeutic use of diacerein for the treatment of osteoarthritis.

### SUMMARY OF THE INVENTION

The present invention therefore provides novel formulations comprising: (a) Diacereine, (b) Meloxicam, (c) one or more anti-adherent agents, (d) one or more disintegrating agents, (e) one or more binder agents, (f) one or more lubricants, (g) one or more diluents, (h) one or more solvents, and (i) any other additive which assists in formulation.

Diacereine is 1,8-diacetoxy-3-carboxy-anthraquinone and it can be represented by the formula (I).

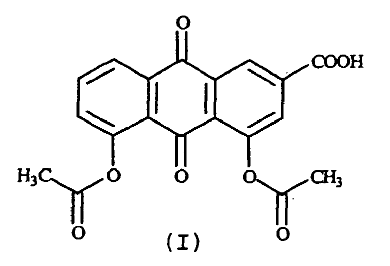

The compound of formula (I) is known by its pharmacological activity as an anti-arthritic, analgesic and anti-inflammatory agent, which acts by inhibitiing Interleukine I which has a predominant role in osteoarthritis. Reine, which is diacereine's active metabolite, is an inhibitor of NADH-associated oxidation, namely it interferes with NADH-dehydrogenase complex function and so with mitocondrial oxidation; it also forms chelates with calcium and copper. Currently is indicated for treatment of osteoarthritis, it is a modifier of cartilage structure and it alleviates pain.

The use of anthraquinone derivatives, as in the case of diacereine, in aqueous solution is not convenient since they are not stable enough to water.

Meloxicam is 4-hydroxy-2-methyl-N-(5-methyl-2-thiazolyl)-2H-1,2-benzothiazine-3-carboxamide-1,1-dioxide and can be represented by formula (II):

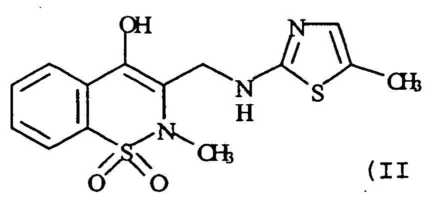

The compound of formula (II) is known by its anti-inflammatory, analgesic and antipyretic activity, and it acts inhibiting the synthesis of prostaglandin with greater potency at the site of inflammation than on kidney and gastric mucosa. Currently is indicated in treatment of rheumatoid arthritis, osteoarthritis, periarthritis in humeral articulation and hip joint, muscular strains, and pain and inflammation in soft tissues and airways with inflammatory processes.

The active ingredient in the formulation is present in an amount in the range from 0.0001% to 95.0% w/w, more preferably from 0.5 to 70.0% w/w for diacereine, and in an amount in the range from 0.0001% to 90.0% w/w, more preferably from 1.0 to 30.0% w/w for Meloxicam.

The present invention is characterized in that the active ingredients combined therein can be present as the anhydrous base or hydrated form or as a physiologically acceptable salt.

### BRIEF DESCRIPTION OF THE DRAWINGS

Figure 1, shows a graph defining the analgesic effects of diacereine/meloxicam combination vs. diacereine and meloxicam individually.
Figure 2, shows a graph of Functionality Index Percentage (FI %) in which the analgesic or antinociceptive effect of diacereine/meloxicam formulation vs. diacereine and meloxicam individually, is described.
Figure 3, shows a graph of analgesic effect 4 hours post-administration of diacereine/meloxicam formulation vs. diacereine and meloxicam individually.

### DETAILED DESCRIPTION OF THE INVENTION

The pharmaceutical formulation can comprise a series of additives or excipients such as, for example, anti-adherents such as colloidal silicon dioxide, calcium sulfate, calcium chloride, talc, corn starch, among others, being the most preferred colloidal silicon dioxide. The anti-adherent can be present in an amount in the range from 0.0001% to 10.0%. Disintegrating agents such as corn starch, alginic acid, cellulose and its derivatives, povidone, sodium crosscarmelose, sodium starch glycolate, among others, the most preferred being sodium crosscarmelose. The disintegrating agent can be present in an amount ranging from 0.0001% to 30.0%. Lubricant agents such as stearic acid, magnesium stearate, talc, among others, the most preferred being talc. The lubricant agent can be present in an amount ranging from 0.0001% to 10.0%. Diluent agents such as lactose, mannitol, calcium phosphate, microcrystalline cellulose, calcium sulfate, sucrose for compression, corn starch, among others, the most preferred being sucrose for compression. The diluent agent can be present in an amount ranging from 1% to 99%. Coatings such as methacrylates, polyvinyl alcohol, derivatives of cellulose, blends of polymers and polysaccharides for coating by film formation utilizing aqueous or organic solvents, with coloring agents, flavoring agents, sugars and any other ingredient used in applications of film forming, coating and making, such as talc, titanium dioxide, among others. Capsules can be hard or soft gelatin capsules, preferably hard gelatin capsules. Polar and non-polar solvents such as water, ethyl alcohol, isopropyl myristate, polyoxypropylenes, propyleneglycol, polyethyleneglycol, glycerol, 70% sorbitol, polyethyleneglycols, mineral oil, petrolatum, lanoline, vegetable waxes, animal waxes, vegetable oils such as olive oil, cottonseed oil, corn oil, among others, preferably ethyl alcohol and polar solvents such as water are used. Final formulation can contain from 1% to 60% w/v of solvent. Binder agents such as polyvidone, tragacanth, acacia, starch, methylcellulose, among others, preferably polyvidone. The binder can be present in an amount ranging from 0.0001% to 20.0%.

Formulations can be contained in blisterpacks made of polyvinyl chloride (PVC) film with a thickness from 200 µ to 250 µ, which can or cannot be coated with polyvinylidene chloride (PVDC) with a weight from 25 g/m² to 120 g/m² and bonded with aluminum foil; they can also be contained in blisterpack made of 2 sheet of aluminum foil bonded together to form the blisterpack, or they can be in cellopolyal film bonded together with aluminum foil. In vials of suitable capacity varying from 5 ml to 500 ml, elaborated from high and/or low density polyethylene, polyethylene terephthalate, polyvinyl chloride, polypropylene, polystyrene, Type I, II, III and IV glass, among others, with or without color. The cap may be of the following types: tamperproof, threaded, cap-to-cap, child proof, elaborated from high and/or low density polyethylene, polyethylene terephthalate, polyvinyl chloride, polypropylene, polystyrene, with or without color. Blisterpack made of polyvinyl chloride coated with polyvinylidene bound to aluminum is preferred.

The elaboration of the different pharmaceutical forms is carried out mixing the active ingredients with the corresponding additives in the adequate concentrations.

Upon administration of diacereine, it undergoes a complete deacetylation process, and it becomes in its active metabolite Reine before it passes to systemic circulation. According to pharmacokinetics studies it binds to proteins in 99%. About 50 to 60% of diacereine orally administered was excreted through kidney as reine and its conjugates

Administering 15 mg of meloxicam every 24 hours will allow to reach peak concentrations of 1.6 µg/ml in plasma. Concentrations at equilibrium can be reached in 3 to 5 days. 99% of meloxicam binds to plasma proteins. The drug has a good distribution in the body, but particularly it achieves high penetration in synovial fluid, reaching levels that are equivalent to the half of concentrations in plasma. Meloxicam is metabolized mainly through oxidation of the methyl group in the thiazolyl molecule. About 50% of the dose is eliminated through urine and the remaining is excreted in feces. The clearance half-life is 20 hours.

It is recommended for treatment of osteoarthritis and related disorders, to initiate with 50 mg of diacereine daily during the first for weeks of treatment, which can prolong for al least 6 months and clinical trials had shown that the drug can be administered to 2 years without having safety problems. For how long should the treatment be continued is a decision taken by the attendant physician. For meloxicam a dose of 15 mg every 24 hours is recommended in adults.

Therefore, the present invention provides a pharmaceutical formulation in solid form, which provides from 5 mg to 150 mg of diacereine and from 1 to 30 mg of meloxicam in suitable doses.

### EXAMPLES

The following examples will assist to illustrate the present invention:

### EXAMPLE 1

| | |
|---|---|
| 1) Diacereine | 23.00% w/w |
| 2) Meloxicam | 7.00% w/w |
| 3) Sodium crosscarmelose | 1.00% w/w |
| 4) Polyvidone k-30 | 1.00% w/w |
| 5) Colloidal silicon dioxide | 0.50% w/w |
| 6) Talc | 3.00% w/w |
| 7) Sucrose for compression | 64.50% w/w |

Tablets are elaborated as follows: meloxicam is mixed with colloidal silicon dioxide, then diacereine and sodium crosscarmelose are added and mixed. Next, polyvidone k-30 is added and mixed. Then sucrose for compression is added and mixed. Finally, the talc is added and mixed. Proceed to tableting.

### EXAMPLE 2

| | |
|---|---|
| 1) Diacereine | 50.00% w/w |
| 2) Meloxicam | 15.00% w/w |
| 3) Sucrose for compression | 24.30% w/w |
| 4) Polyvidone k-30 | 3.00% w/w |
| 5) Colloidal silicon dioxide | 0.50% w/w |
| 6) Sodium crosscarmelose | 5.00% w/w |
| 7) Talc | 2.20% w/w |

Powder elaboration is carried out as follows: Diacereine is mixed with colloidal silicon dioxide, to this meloxicam and sodium crosscarmelose are added and mixed. Then polyvidone k-30 and sucrose for compression are added and mixed. Finally, talc is added and mixed. Proceed to encapsulation.

Next, the clinical study of meloxicam/diacereine combination is provided, which is carried out by oral route.

### Determination of potency, effectiveness and type of analgesic sinergism produced by blend of meloxicam + diacereine orally administered, on artrithis

### Pre-clinical study of diacereine/meloxicam blend

### Antecedents

A great number of non-steroidal anti-inflammatory analgesics (NSAIA) are generally used for treatment of osteoarthritis as a first line therapy, however, their clinical activity is limited by several side effects. Osteoarthritis is a degenerative process of joints, characterized by cartilage progressive destruction and erosion, and it is associated with age. The articular cartilage degenerates and eventually is worn out the bone surface, this and subsequent changes produce pain and limitations on the movement of affected patients. NSAIA's provide general symptomatic alleviation in patients affected with osteoarthritis, but those agents decrease prostaglandin synthesis. Unlike NSAIA's, diacereine can stimulate or not affect prostaglandin synthesis and it acts by inhibiting Interleukin-1, therefore it has a potential use as an alternative.

It is believed that inflammatory mediators such as Interleukin-1 (IL-1) play an important role in joint diseases such as osteoarthritis and rheumatoid arthritis. Diacereine, an anthraquinone derivative, is a compound with an unusual mechanism of action, it is an inhibitor of IL-1 that exhibits analgesic, antipyretic and anti-inflammatory activity, affecting pain threshold in rats affected with limb edema and on hyperpyrexia on rabbits (Kay et al., 1980). Diacereine and its metabolite (reine) inhibit Interleukin-1 beta production in human monocytes in vitro (Berdah et al., 1993). Although it inhibits IL-1 that mediates colagenase production in articular cartilage (Boittin et al., 1993), it does not disrupt the activity of cyclooxygenase (COX) from kidney or platelet (LaVilla et al., 1989).

The association of diacereine/meloxicam is an association that has the potential of therapeutic use in patients affected with disorders such as osteoarthritis and rheumatoid arthritis. It is expected, by virtue of the type of analgesic that meloxicam is (mainly peripheral action), a suitable analgesic synergism when it is associated with diacereine (an active agent with mode of action different to prostaglandin inhibition).

### Procedure

The experiments were carried out using male Wistar rats having a body weight between 180 and 200 g. All of the experimental procedures were carried out following recommendations from The Committee for Research and Ethical Issues of the International Association for the Study of Pain (Covino et al., 1980) and Guidelines on Ethical Standards for Investigations of Experimental Pain in animals (Zimmermann 1983). The number of test animals was taken to the minimal (6 animals for each experimental aspect). Animals were kept in a room with light/darkness cycles. Rats were fastened twelve hours prior the beginning of experiments, leaving only free access to water. All the experiments were run during the light phase, using the animals only once.

### Evaluation of Analgesic activity

The evaluation of analgesic effects was carried out using the Pain Induced Functional Impairment model in the Rat (PIFIR) model. Animals were anesthetized in a glass desiccator saturated with ether vapors. Gout arthritis was induced, upon application of a intra-articular injection of 0.05 ml of uric acid suspended in mineral oil, in the right-side hind leg, exactly in the femoral-tibio-patellar joint. Immediately after, an electrode was attached to each hind leg in the middle of each pad. Rats were allowed to recover from anesthesia and afterwards were placed in a rotating stainless steel cylinder of 30 cm in diameter. The cylinder was rotated at 4 rpm, forcing the rats to walk for 2 minutes every 15 minutes during the following hour, and every half an hour, for 3 more hours, for a total of 4 hours after onset of total dysfunction. Variable measured was contact time of each hind leg of every rat in the cylinder.

### Experimental protocol

Analgesic effects produced by meloxicam, diacereine or meloxicam/diacereina blend were determined and analyzed individually once gout arthritis was established in rats. Rats were administered with each of the compounds orally. From the moment when the uric acid was administered in the joint to produce a gout type dysfunction and alteration, 2.5 hours were allowed to pass in order to produce total dysfunction (gout arthritis was already complete 2.5 hours after uric acid was administered). This period of time (2.5 hours after administration of uric acid) was considered as time "0", in order to administer in this point of time the antinociceptive or analgesic treatment and the temporary course of each treatment were determined during the following 4 continuous hours. For each treatment a "n" value of 6 was utilized.

### Data disclosure

Data are expressed as Percentage of Functionality Index (FI %). This FI % is the ratio obtained dividing contact time of the limb with uric acid by the contact time of the contralateral limb of the same rat and this is multiplied by 100. Temporal course(TC) curves are constructed when graphing FI% vs time (h); the analgesic or antinociceptive effect was estimated as FI% recovery. The accumulated analgesic effect during the total observation period (4 h), was determined as area under curve (AUC) of TC, using trapezoidal rule (Rowland and Toser, 1989), and it also was used to construct dose-response curves (DRC).

### Results and conclusions

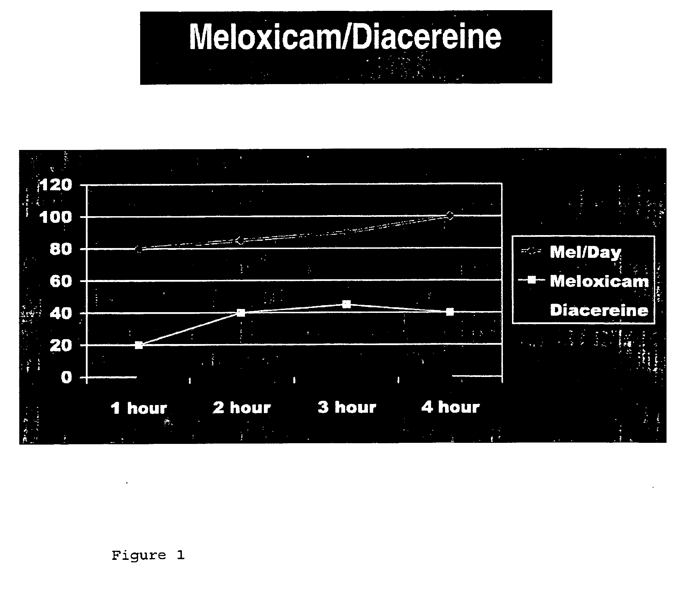

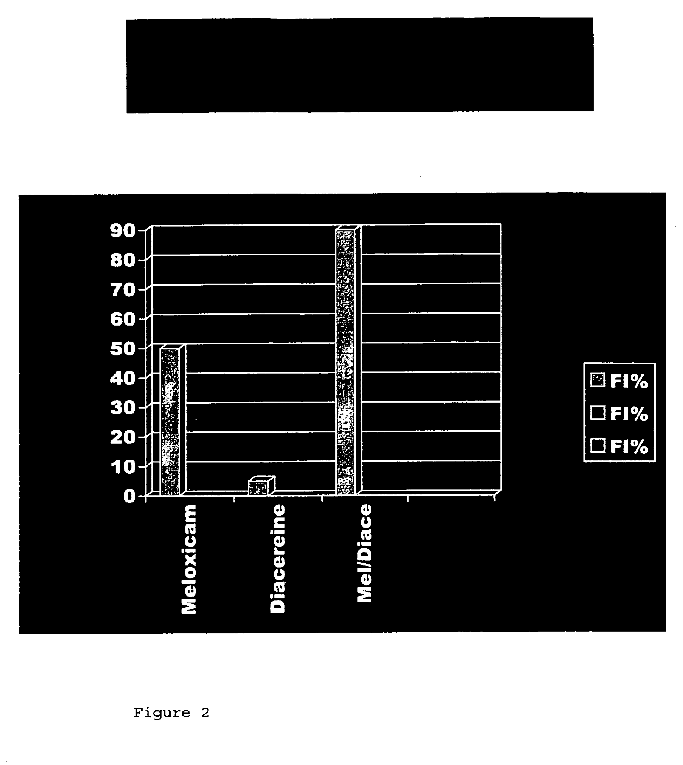

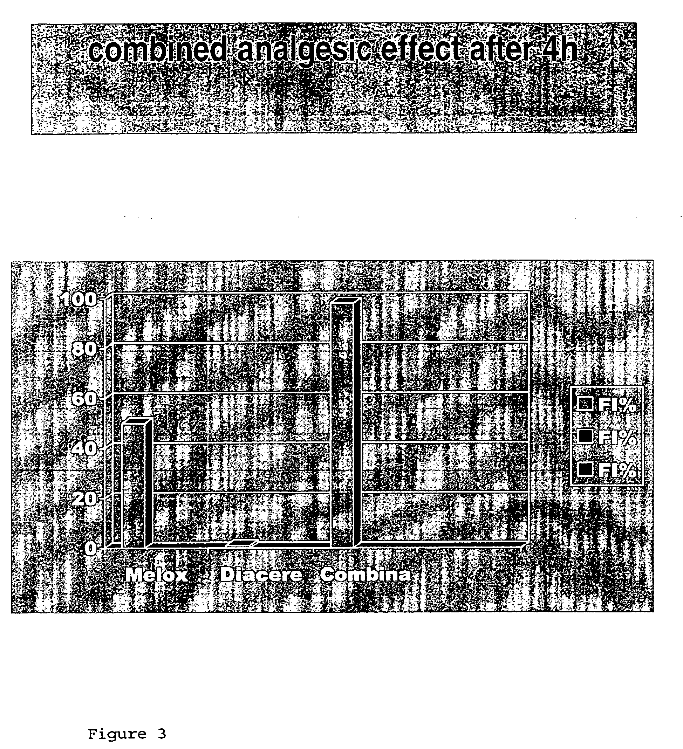

1. As for TC efficacy: Diacereine was unable to generate analgesic effects (Fig. 1), whereas the blend of Diacereine/meloxicam exhibit superior efficacy compared to that showed by meloxicam when administered in single doses.
2. As for the onset of action: Meloxicam generated its effect, in general, in a more delayed manner.
3. Regarding duration of analgesic effect: analgesic effects were analyzed during 4 continuous hours: meloxicam and the blend tend to maintain the Emax obtained up to the 4 continuous hours.
4. As for global analgesic efficacy (GAE) evaluated during 4 continuous hours: The blend exhibits greater efficacy compared to meloxicam (Fig. 2).
5. As for analgesic potency, meloxicam was more potent than diacereine alone, but DRC's showed that the blend was more potent than meloxicam (Fig. 3).

Under the experimental conditions and pain established in the PIFIR model, the blend of diacereine/meloxicam produced an important improvement in analgesic efficacy an therapeutic coverage compared to the single administration of meloxicam. Combining small doses it is possible to overcome the efficacy of meloxicam.

## Claims

1. A solid pharmaceutical formulation wherein said formulation comprises the synergistic combination of Diacereine, and meloxicam, as well as pharmaceutically acceptable excipients, in particular (a) one or more anti-adherent agents, (b) one or more disintegrating agents, (c) one or more binding agents, (d) one or more lubricating agents, (e)one or more diluting agents, (f) one or more solvents, and (g) any other additive which assists in formulation.

2. Solid pharmaceutical formulation according to claim 1, wherein the concentration of the two active agents, that is Diacerein and Meloxicam, is from 0.0001% to 95.0% w/w, preferably from 0.5 to 70.0% w/w for Diacereine, and from 0.0001% to 90.0% w/w, preferably from 1.0 to 30.0% w/w for Meloxicam.

3. Solid pharmaceutical formulation according any one of the claims 1-2, wherein the solid pharmaceutical form contains a series of pharmaceutically acceptable additives or excipients selected from the group consisting of: (i) anti-adherents agents, such as colloidal silicon dioxide, calcium sulphate, , calcium chloride, talc, corn starch, among others, with colloidal silicon dioxide being preferred, which can be present in the formulation in an amount from 0.0001% to 10.0%, (ii) disintegrating agents such as corn starch, alginic acid, celluloses and its derivatives, povidone, sodium crosscarmelose, sodium starch glycolate, among others, with sodium crosscarmelose being preferred, which can be present in the formulation in an amount from 0.0001% to 30.0%; (iii) lubricating agents selected from the group consisting of stearic acid, magnesium stearate, talc, among others, with talc being preferred, which can be present in the formulation in an amount from 0.0001% to 10.0%, (iv) diluting agents such as lactose, mannitol, calcium phosphate, microcrystalline cellulose, calcium sulfate, sucrose for compression, corn starch, among others, with sucrose for compression being preferred, which can be present in the formulation in an amount from 1% to 99%, and (v) binding agents selected from the group consisting of polyvidone, tragacanth, acacia, starch, methylcellulose, among others with polyvidone being preferred, which can be present in the formulation in an amount from 0.0001% to 20.0%

4. A solid pharmaceutical formulation according to any one of the claim 1-3, wherein the solid pharmaceutical form can contain, in addition, other components, such as: coating agents selected from the group consisting of methacrylates, polyvinyl alcohol, cellulose derivatives, blends of polymers and polysaccharides for film formation using aqueous or organic solvents, coloring agents, flavoring agents, sugars and any other ingredient that can be used in film forming, coating and dragee-making applications.

5. Solid pharmaceutical formulation according to any one of the claims 1-4, wherein the solid pharmaceutical formulation may contain one or more polar and non-polar solvents selected from the group consisting of: water, ethyl alcohol, isopropyl myristate, polyoxypropylenes, propylene glycol, polyethylene glycol, glycerol, sorbitol solution, among others, wherein the final formulation contains from 1% to 60% w/v of solvent.

6. Solid pharmaceutical formulation according to any one of the calms 1-5, wherein the combined active agents contained therein, can be present as the anhydrous or hydrated base or as a physiologically acceptable salt, preferably, Diacerein base for Diacerein, and Meloxicam base for Meloxicam.

7. Solid pharmaceutical formulation according to any one of the claims 1-6, wherein it comprises from 5 mg to 150 mg of Diacerein and from 1 to 30 mg of Meloxicam in suitable doses.

8. Solid pharmaceutical formulation according to any one of the claims 1-7, wherein said formulation is formulated to be administered orally in the form of a tablet and capsule.

9. Solid pharmaceutical formulation according to claim 8 wherein said capsules or tablets are contained in a blisterpack made of a polyvinyl chloride (PVC) film with a thickness from 200 µ to 250 µ, which can be uncoated or coated with polyvinylidene chloride (PVDC) having a weight from 25 g/m² to 120 g/m² and bonded with aluminum foil, said capsules or tablets can also be contained in blisterpack, made of from two sheet of aluminum foil bonded together to form said blister, or as an alternative, cellopolyal film with aluminum foil bonded together.

10. Solid pharmaceutical formulation according to claim 8, wherein said capsules or tablets can be contained in vials of suitable capacity varying from 5 ml to 500 ml, made of high and/or low density polyethylene, polyethylene terephthalate, polyvinyl chloride, polypropylene, polystyrene, Type I, II, III and IV glass, among others, with or without color.

11. Use of the solid pharmaceutical formulation according to any one of the claims 1-10, in the preparation of a medicament for treatment of osteoarthritis, rheumatoid arthritis, gout arthritis, multiple sclerosis, amyotrophic lateral sclerosis and related diseases, in addition of inflammatory processes originated from various etiologies, by administering suitable doses.

## Patentansprüche

1. Feste pharmazeutische Formulierung, wobei die Formulierung die synergistische Kombination aus Diacerein und Meloxicam umfasst, wie auch pharmazeutisch annehmbare Hilfsstoffe, insbesondere (a) ein oder mehrere Antihaftmittel, (b) ein oder mehrere Zersetzungsmittel, (c) ein oder mehrere Bindemittel, (d) ein oder mehrere Schmiermittel, (e) ein oder mehrere Verdünnungsmittel, (f) ein oder mehrere Lösemittel, und (g) jeden anderen Zusatzstoff, der zur Formulierung beiträgt.

2. Feste pharmazeutische Formulierung nach Anspruch 1, wobei die Konzentration der zwei Wirkstoffe, das heißt, Diacerein und Meloxicam, von 0,0001% bis 95,0% w/v reicht, vorzugsweise von 0,5 bis 70,0% w/w für Diacerein und von 0,0001% bis 90,0% w/w, vorzugsweise von 1,0 bis 30,0% w/w, für Meloxicam.

3. Feste pharmazeutische Formulierung nach einem der Ansprüche 1 bis 2, wobei die feste pharmazeutische Form eine Reihe von pharmazeutisch annehmbaren Zusatzstoffen oder Hilfsstoffen enthält, die ausgewählt sind aus der Gruppe bestehend aus: (i) Antihaftmitteln, wie kolloidalem Siliziumdioxid, Kalziumsulfat, Kalziumchlorid, Talk, Maisstärke, unter anderen, wobei kolloidales Siliziumdioxid bevorzugt ist, die in der Formulierung in einer Menge von 0,0001% bis 10,0% vorhanden sein können, (ii) Zersetzungsmitteln, wie Maisstärke, Alginsäure, Zellulosen und deren Derivate, Povidon, Croscarmelose-Natrium, Natriumstärkeglykolat, unter anderen, wobei Croscarmelose-Natrium bevorzugt ist, die in der Formulierung in einer Menge von 0,0001% bis 30,0% vorhanden sein können; (iii) Schmiermitteln, die ausgewählt sind aus der Gruppe bestehend aus Stearinsäure, Magnesiumstearat, Talk, unter anderen, wobei Talk bevorzugt ist, die in der Formulierung in einer Menge von 0,0001% bis 10,0% vorhanden sein können; (iv) Verdünnungsmitteln, wie Laktose, Mannitol, Kalziumphosphat, mikrokristalline Zellulose, Kalziumsulfat, Sukrose zur Kompression, Maisstärke, unter anderen, wobei Sukrose zur Kompression bevorzugt ist, die in der Formulierung in einer Menge von 1% bis 99% vorhanden sein können, und (v) Bindemitteln, die ausgewählt sind aus der Gruppe bestehend aus Polyvidon, Tragakanth, Akazie, Stärke, Methylzellulose, unter anderen, wobei Polyvidon bevorzugt ist, die in der Formulierung in einer Menge von 0,0001% bis 20,0% vorhanden sein können.

4. Feste pharmazeutische Formulierung nach einem der Ansprüche 1 bis 3, wobei die feste pharmazeutische Form zusätzlich andere Komponenten enthalten kann, wie: Beschichtungsmittel, die ausgewählt sind aus der Gruppe bestehend aus Methacrylaten, Polyvinylalkohol, Zellulosederivaten, Mischungen aus Polymeren und Polysacchariden zur Filmbildung unter Verwendung wässeriger oder organischer Lösemittel, Farbstoffen, Geschmacksstoffen, Zuckern und anderen Inhaltsstoffen, die in Anwendungen zur Filmbildung, Beschichtung und Drageeherstellung verwendet werden können

5. Feste pharmazeutische Formulierung nach einem der Ansprüche 1 bis 4, wobei die feste pharmazeutische Formulierung ein oder mehr polare und nicht polare Lösemittel enthalten kann, die ausgewählt sind aus der Gruppe bestehend aus: Wasser, Ethylalkohol, Isopropylmyristat, Polyoxypropylenen, Propylenglykol, Polyethylenglykol, Glycerol, Sorbitollösung, unter anderen, wobei die Endformulierung 1% bis 60% w/v Lösemittel enthält.

6. Feste pharmazeutische Formulierung nach einem der Ansprüche 1 bis 5, wobei die darin enthaltenen, kombinierten Wirkstoffe als wasserfreie oder hydrierte Basis oder als physiologisch annehmbares Salz vorhanden sein können, vorzugsweise als Diacereinbasis für Diacerein und Meloxicambasis für Meloxicam.

7. Feste pharmazeutische Formulierung nach einem der Ansprüche 1 bis 6, wobei diese 5 mg bis 150 mg Diacerein und 1 bis 30 mg Meloxicam in geeigneten Dosen umfasst.

8. Feste pharmazeutische Formulierung nach einem der Ansprüche 1 bis 7, wobei die Formulierung zur oralen Verabreichung in der Form einer Tablette oder Kapsel formuliert ist.

9. Feste pharmazeutische Formulierung nach Anspruch 8, wobei die Kapseln oder Tabletten in einer Blisterpackung enthalten sind, die aus einem Polyvinylchlorid-(PVC-) Film mit einer Stärke von 200 µ bis 250 µ besteht, der unbeschichtet oder mit Polyvinylidenchlorid (PVDC), mit einem Gewicht von 25 g/m² bis 120 g/m², beschichtet und an Aluminiumfolie gebunden sein kann, wobei die Kapseln oder Tabletten auch in einer Blisterpackung enthalten sein können, die aus zwei Schichten Aluminiumfolie besteht, die zur Bildung des Blisters aneinander gebunden sind, oder als Alternative, Cellopolyalfilm mit Aluminiumfolie, die aneinander gebunden sind.

10. Feste pharmazeutische Formulierung nach Anspruch 8, wobei die Kapseln oder Tabletten in Ampullen geeigneter Kapazität enthalten sein können, die von 5 ml bis 500 ml variiert, die aus einem Polyethylen hoher und/oder geringer Dichte, Polyethylenterephthalat, Polyvinylchlorid, Polypropylen, Polystyrol, Typ I, II, III und IV Glas, unteren anderen, mit oder ohne Farbe bestehen.

11. Verwendung der festen pharmazeutischen Formulierung nach einem der Ansprüche 1 bis 10 in der Herstellung eines Medikaments zur Behandlung von Osteoarthritis, rheumatoider Arthritis, Gichtarthritis, Multipler Sklerose, amyothropher Lateralsklerose und verwandten Erkrankungen, zusätzlich zu entzündlichen Prozessen, die ihren Ursprung in verschiedenen Ätiologien haben, durch Verabreichung geeigneter Dosen.

## Revendications

1. Formulation pharmaceutique solide, ladite formulation comprenant la combinaison synergique de diacéréine et de méloxicam ainsi que des excipients pharmaceutiquement acceptables, en particulier (a) un ou plusieurs agents anti-adhésifs, (b) un ou plusieurs agents de décomposition, (c) un ou plusieurs agents de liaison, (d) un ou plusieurs agents lubrifiants, (e) un ou plusieurs agents de dilution, (f) un ou plusieurs solvants, et (g) tout autre additif qui aide à la formulation.

2. Formulation pharmaceutique solide selon la revendication 1, dans laquelle la concentration des deux agents actifs que sont la diacéréine et le méloxicam, est de 0,0001 % à 95,0 % p/p, de préférence, de 0,5 à 70,0 % p/p pour la diacéréine et de 0,0001 % à 90,0 % p/p, de préférence de 1,0 à 30,0 % p/p pour le méloxicam.

3. Formulation pharmaceutique solide selon l'une quelconque des revendications 1 à 2, la forme pharmaceutique solide contenant une série d'additifs ou d'excipients pharmaceutiquement acceptables choisis dans le groupe comprenant : (i) des agents anti-adhésifs tels que le dioxyde de silicium colloïdal, le sulfate de calcium, le chlorure de calcium, le talc, l'amidon de maïs, entre autres, le dioxyde de silicium colloïdal étant préféré, qui peuvent être présents dans la formulation en une quantité de 0,0001 % à 10,0 %, (ii) des agents de décomposition, tels que l'amidon de maïs, l'acide alginique, les celluloses et leurs dérivés, la povidone, la croscarmellose sodium, le glycolate d'amidon sodium, entre autres, la croscarmellose sodium étant préférée, qui peuvent être présents dans la formulation en une quantité de 0,0001 % à 30,0 % ; (iii) des agents lubrifiants choisis dans le groupe comprenant l'acide stéarique, le stéarate de magnésium, le talc, entre autres, le talc étant préféré, qui peuvent être présents dans la formulation en une quantité de 0,0001 % à 10,0 %, (iv) des agents de dilution tels que le lactose, le mannitol, le phosphate de calcium, la cellulose microcristalline, le sulfate de calcium, la saccharose pour compression, l'amidon de maïs, entre autres, la saccharose pour compression étant préférée, qui peuvent être présents dans la formulation en une quantité de 1 % à 99 %, et (v) des agents de liaison choisis dans le groupe comprenant la polyvidone, la gomme adragante, la gomme arabique, l'amidon, la méthylcellulose, entre autres, la polyvidone étant préférée, qui peuvent être présents dans la formulation en une quantité de 0,0001 % à 20,0 %.

4. Formulation pharmaceutique solide selon l'une quelconque des revendications 1 à 3, la forme pharmaceutique solide pouvant contenir de plus, d'autres composants tels que des agents de revêtement choisis dans le groupe comprenant les méthacrylates, l'alcool de polyvinyle, les dérivés de cellulose, les mélanges de polymères et de polysaccharides pour la formation d'un film en utilisant des solvants aqueux ou organiques, des agents colorants, des agents aromatisants, des sucres et tout autre ingrédient qui peut être utilisé dans la formation de film, le revêtement et la production de dragées.

5. Formulation pharmaceutique solide selon l'une quelconque des revendications 1 à 4, la formulation pharmaceutique solide pouvant contenir un ou plusieurs solvants polaires ou non polaires choisis dans le groupe comprenant l'eau, l'alcool d'éthyle, le myristate d'isopropyle, les polyoxypropylènes, le propylène glycol, le polyéthylène glycol, le glycérol, une solution de sorbitol, entre autres, la formulation finale contenant de 1 % à 60 % p/v de solvant.

6. Formulation pharmaceutique solide selon l'une quelconque des revendications 1 à 5, dans laquelle les agents actifs combinés contenus dans celle-ci peuvent être présents en tant que base anhydre ou hydratée ou en tant que sel physiologiquement acceptable, de préférence, la diacéréine base pour la diacéréine et le méloxicam base pour le méloxicam.

7. Formulation pharmaceutique solide selon l'une quelconque des revendications 1 à 6, comprenant de 5 mg à 150 mg de diacéréine et de 1 à 30 mg de méloxicam en doses appropriées.

8. Formulation pharmaceutique solide selon l'une quelconque des revendications 1 à 7, ladite formulation est formulée de façon à être administrée oralement sous la forme d'un comprimé et d'une capsule.

9. Formulation pharmaceutique solide selon la revendication 8, lesdites capsules ou lesdits comprimés étant contenu(e)s dans un emballage thermoformé constitué d'un film de chlorure de polyvinyle (PVC) présentant une épaisseur de 200 µm à 250 µm qui peut être non revêtu ou revêtu avec du chlorure de polyvinylidène (PVDC) ayant un poids de 25 g/m² à 120 g/m² et lié à une feuille d'aluminium, lesdites capsules ou lesdits comprimés pouvant également être contenus dans l'emballage thermoformé, constitué de deux feuilles d'aluminium liées conjointement pour former ledit emballage thermoformé ou en variante, un film cellophane avec une feuille d'aluminium liés conjointement.

10. Formulation pharmaceutique solide selon la revendication 8, lesdites capsules ou lesdits comprimés pouvant être contenus dans des flacons de capacité appropriée variant de 5 ml à 500 ml, constitués de polyéthylène de haute densité et/ou de basse densité, de polyéthylène téréphtalate, de chlorure de polyvinyle, de polypropylène, de polystyrène, de verre de type I, II, III et IV, entre autres, avec ou sans couleur.

11. Utilisation de la formulation pharmaceutique solide selon l'une quelconque des revendications 1 à 10, dans la préparation d'un médicament pour le traitement de l'arthrose, de l'arthrite rhumatoïde, de l'arthrite goutteuse, de la sclérose en plaques, de la sclérose latérale amyotrophique et des maladies apparentées, en plus des processus inflammatoires d'étiologies diverses, par l'administration de doses appropriées.
